Europäisches Patentamt

European Patent Office

Office européen des brevets

(11) Veröffentlichungsnummer: **0 159 041 B1**

# EUROPÄISCHE PATENTSCHRIFT

(45) Veröffentlichungstag der Patentschrift: 27.02.91

(51) Int. Cl.⁵: **A61B 5/0235, F16K 7/06**

(21) Anmeldenummer: 85104730.8

(22) Anmeldetag: 18.04.85

(54) Ablassventil für Blutdruckmessgeräte und dergleichen.

(30) Priorität: 18.04.84 DE 3414709

(43) Veröffentlichungstag der Anmeldung:
23.10.85 Patentblatt 85/43

(45) Bekanntmachung des Hinweises auf die
Patenterteilung:
27.02.91 Patentblatt 91/09

(84) Benannte Vertragsstaaten:
**AT CH FR GB IT LI**

(56) Entgegenhaltungen:
**DE-B- 2 747 060**
**DE-U- 8 412 226**
**FR-A- 2 239 230**
**US-A- 2 660 395**

(73) Patentinhaber: **Speidel + Keller GmbH + Co.
KG**
**Zollernstrasse 2**
**D-7455 Jungingen(DE)**

(72) Erfinder: **Speidel, Blasius**
**Hochmeisterstrasse 33**
**D-7455 Jungingen(DE)**

(74) Vertreter: **Kastner, Hermann, Dipl.-Ing.**
**Osterholzallee 89**
**D-7140 Ludwigsburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der Blutdruckmessung nach Riva-Rocci und Korotkoff wird eine aufblasbare Meßmanschette um eine Extremität der Untersuchungsperson herumgelegt und mittels eines Drucklufterzeugers die Meßmanschette über den zu erwartenden systolischen Blutdruckwert hinaus aufgepumpt. Anschließend muß der Innendruck der Meßmanschette möglichst feinfühlig und möglichst gleichmäßig erniedrigt werden, damit zunächst beim Erreichen des systolischen Blutdruckwertes das Einsetzen der Korotkoff'schen Strömungsgeräusche akustisch wahrgenommen werden kann und damit später beim diastolischen Blutdruckwert das Verschwinden der Strömungsgeräusche wahrgenommen werden kann. Zu diesem Zweck ist in die Verbindungsleitung zwischen der Meßmanschette und dem Druckerzeuger ein Ablaßventil eingeschaltet, das eine Dosiereinrichtung für die über eine Auslaßöffnung ausströmenden Druckluft aufweist.

Unter den bekannten Ablaßventilen gibt es solche mit einer Ablaßschraube, die ein kegeliges Ende als Ventilkörper aufweist, der mit einem hohlkegeligen Ventilsitz zusammenwirkt. Bei dieser Art Ablaßventil stört vor allem die ergonomisch ungünstige Betätigungsbewegung, die von der Bedienungsperson eine beträchliche Fingerfertigkeit und Geschicklichkeit erfordert. Andere bekannte Ablaßventile weisen als Ventilkörper einen schlanken Ventilkegel auf, der mit einem zylindrischen oder einem ebenfalls kegeligen Ventilsitz zusammenwirkt. Der Ventilkegel wird entweder durch eine Schiebetaste oder durch eine Schwenktaste betätigt. Beide Ausführungsformen haben den Nachteil, daß bei der Öffnungsbewegung des Ventilkegels, die im allgemeinen linear erfolgt, die Querschnittsfläche einer quadratischen Funktion folgt. Die dadurch bedingte verhältnismäßig starke Zunahme der Querschnittsfläche hat zur Folge, daß bei unterschiedlicher Betätigung des Ventilkegels, wie sie zum Beispiel im Laufe der Abnahme des Innendruckes oder bei unterschiedlichem Anfangsdruck erforderlich ist, unterschiedlich große Ablaßgeschwindigkeiten auftreten. Dadurch ist die Feinfühligkeit nicht über den gesamten Betätigungsbereich gleich gut.

Es ist ein Ablaßventil bekannt (DE- A 32 41 939), bei dem das Ventilorgan durch zwei aus einem gummielastischen Werkstoff hergestellte Platten gebildet wird, zwischen denen ein starres Glied angeordnet ist, das einen Spalt mit einem gewissen Durchlaßquerschnitt offenhält. Da die beiden Platten auf ihrer Außenseite dem Überdruck ausgesetzt sind, werden sie umso stärker gegeneinander gedrückt, je größer der Überdruck ist. Daher ist der Spaltquerschnitt bei höherem Überdruck kleiner und vergrößert sich bei abnehmendem Überdruck. Dadurch kann in gewissen Grenzen eine bessere Linearität des Druckabfalls erreicht werden als bei unveränderlicher Drosselöffnung. Dieses Ablaßventil läßt sich aber nicht betätigen. Es kann also nur dort eingesetzt werden, wo es auf eine Beeinflussung des Druckabfalls nicht ankommt.

In ähnlicher Weise arbeitet ein anderes bekanntes Ablaßventil (DE-A 27 59 119), bei dem das Ventilorgan durch einen gummielastischen Hohlkörper gebildet wird, der in seiner Wand wenigstens einen Schlitz aufweist, durch den ein starrer stiftförmiger Körper hindurchgesteckt ist. Auch hier drückt der den Hohlkörper außen umgebende Überdruck die Wand in der Nachbarschaft des Stiftes umso stärker zusammen, je höher der Überdruck ist. Auch dieses Ablaßventil läßt sich nicht betätigen.

Der Erfindung liegt die Aufgabe zugrunde, ein betätigbares Ablaßventil zu schaffen, das über den gesamten Betätigungsweg eine sehr feinfühlige und weitgehend gleichmäßige Dosierung der Druckreduzierung ermöglich.

Diese Aufgabe wird durch ein Ablaßventil mit den im Anspruch 1 oder im Anspruch 8 angegebenen Merkmalen gelöst.

Bei der Ausführungsform nach Anspruch 1 wird der zwischen den beiden Schlauchstutzen in einem freien Bogen verlaufende Ventilschlauch vom Druckkörper unter der Wirkung der Schließkraft in seiner Bogenebene eingedrückt. Gleichzeitig wird die am Bogen außen gelegene Schlauchwand in der Querschnittsebene senkrecht zur Bogenebene eingebeult und gegen die am Bogen innen gelegene Schlauchwand gedrückt, so daß an dieser Stelle der Hohlraum des Schlauches vollständig geschlossen wird. Beim Betätigen der Betätigungstaste wird der Druckkörper entgegen der Schließkraft mehr oder minder weit vom Schlauch abgehoben, so daß sowohl die außen gelegene Schlauchwand von der innen gelegenen Schlauchwand mehr oder minder weit abgehoben wird wie auch der in der Bogenebene eingedrückte Schlauch mehr oder minder weit zu seiner ursprünglichen Bogenform zurückkehrt. Dadurch wird der Hohlraum des Schlauches in entsprechendem Maße mehr oder minder weit geöffnet, so daß die Luft aus dem Luftkanal in dem gewünschten Maße entweichen kann. Da beim Ventilschlauch an der Einwirkungsstelle des Druckkörpers die am Schlauchbogen außen gelegene Schlauchwand über die Einebnung hinweg eingebeult wird und seine Querschnittsform ebenso wie seine Bogenform eine negative Wölbung erfährt, bildet die auf der Innenseite des Schlauchbogens gelegene Schlauchwand eine Art Wanne mit räumlich gekrümmter Oberfläche, an die sich die außen gelegene Schlauchwand sehr eng anschmiegt. Infolge dieser räumlich starken elastischen Verformung der Schlauchwandbereiche ergibt sich einerseits in der Schließstellung des Druckkörpers eine gute Abdichtung im Ventilschlauch und andererseits ein verhältnismäßig weiter Verformungsweg der eingebeulten Schlauchwandbereiche, der vor allem am Anfang der Rückkehrbewegung zur normalen Form von einer weitgehend parallelen Verformung der beiden aufeinanderliegenden Schlauchwandbereiche begleitet wird. Dadurch erfolgt das erste Öffnen eines Luftdurchlasses und das anschließende weitere Öffnen dieses Luftdurchlasses nur sehr allmählich. Dadurch wird eine sehr feinfühlige Dosierung der Druckreduzierung erreicht, die über einen wünschenswerten weiten Betätigungsweg des Druckkörpers hin erhalten bleibt. Darüber hinaus nimmt die Vergrößerung des Luftdurchlasses später aber um so stärker zu, so daß nach dem Erfassen des diastolischen Blutdruckwertes die wünschenswert schnelle und gründliche Entlüftung der Meßmanschette ebenso einfach zu bewerkstelligen ist.

Bei der Ausführungsform nach Anspruch 8 stellen sich sehr ähnliche Verhältnisse ein, wobei hier der Ventilschlauch durch den balligen Druckkörper in die Vertiefung der Rinne hineingedrückt wird. Er verformt sich dabei sowohl bei der Schließbewegung wie auch bei der Öffnungsbewegung in ähnlicher Weise wie es zuvor geschildert wurde.

Wenn ein Ablaßventil in der Ausführungsform nach Anspruch 1 nach Anspruch 2 ausgestaltet wird, ergeben sich besonders günstige Verhältnisse hinsichtlich des Verformungsverhaltens der beteiligten Wandbereiche des Ventilschlauches. Das gleiche gilt für ein nach Anspruch 3 ausgestalteten Ablaßventil. Bei einer Ausgestaltung des Ablaßventils nach Anspruch 4 ist durch die Schwenktaste eine leichte Bedienbarkeit des Ablaßventils und seines Druckkörpers gegeben, wobei zugleich eine anatomisch günstige Lage und Ausrichtung der Tastfläche und ein entsprechend günstiger Bewegungsablauf erreicht werden. Außerdem wird dadurch eine Weguntersetzung von der Tastfläche zum Druckkörper hin erreicht. Aufgrund des größeren Betätigungsweges der Tastfläche gegenüber dem Betätigungsweg des Druckkörpers wird nochmals eine Verbesserung der Feinfühligkeit bei der Dosierung erreicht. Bei der Ausgestaltung des Ablaßventils nach Anspruch 5 wird die Herstellung der Feder sehr einfach und sehr billig. Außerdem kann sie einfach und leicht montiert werden, indem sie vor dem Anbringen der Schwenktaste einfach mit dem einen Schenkel in die Ausnehmung des Ventilgehäuses eingelegt wird und darin nach dem Aufsetzen der Schwenktaste durch die dabei sich einstellende Vorspannung festgehalten wird. Bei einer Ausgestaltung des Ablaßventils nach Anspruch 6 wird erreicht, daß der Ventilschlauch leichter und schneller in seine Ausgangsform zurückkehrt, wenn der Druckkörper von ihm abgehoben wird. Das gilt insbesondere für den Fall, daß das Ablaßventil aus Versehen oder absichtlich längere Zeit geschlossen gehalten wurde, und dadurch die elastische Eigenspannung des Ventilschlauchs sich etwas vermindert hat. Das gleiche wird bei einer Ausgestaltung des Ablaßventils nach Anspruch 7 erreicht.

Wenn das Ablaßventil in der Ausführungsform nach Anspruch 8 gemäß Anspruch 9 ausgestaltet wird, wird eine besonders gute Dichtwirkung der Ventilteile erreicht. Bei einer Ausgestaltung nach Anspruch 10 ergeben sich die gleichen Vorteile wie bei der entsprechenden Ausgestaltung des Ablaßventils in der Ausführungsform nach Anspruch 1. Bei einer Ausgestaltung des Ablaßventils nach Anspruch 11 ergibt sich eine einfache Herstellung und eine ebenso einfach Führung für den Druckkörper. Bei einer Ausgestaltung des Ablaßventils nach Anspruch 12 ergeben sich besonders günstige Verformungs- und Bewegungsverhältnisse. Bei einer Ausgestaltung des Ablaßventils nach Anspruch 13 wird einerseits eine gute Lagesicherung für den Ventilschlauch geschaffen, durch die gewährleistet ist, daß die miteinander zusammenwirkenden Teile auch bei ungünstigen Betriebsverhältnissen oder ungeschickter Handhabung ihre richtige Zuordnung zueinander beibehalten. Andererseits wird durch den abnehmbaren Deckel die Herstellung aller Teile und ihre Montage erleichtert. Das gilt sowohl für die Erstmontage wie auch für spätere Reparatur- und Wartungsarbeiten, wenn etwa infolge einer Werkstoffalterung der Ventilschlauch ersetzt werden muß.

Bei einer Ausgestaltung des Ablaßventils nach Anspruch 14 oder 15 wird der Ventilschlauch elastisch gestreckt. Dadurch tritt er beim Zurückweichen des Druckkörpers leichter und schneller aus der Vertiefung in der Rinne heraus, wobei auch der dem Druckkörper benachbarte oben gelegene Wandbereich den Stellbewegungen des Druckkörpers ohne Zeitverzögerung, daß heißt ohne zeitlichen und auch räumlichen Nachlauf, folgt.

Durch eine Ausgestaltung des Ablaßventils nach Anspruch 16 können die ohnehin günstigen Verformungsverhältnisse des Ventilschlauchs und die Betätigungsverhältnisse an der Betätigungstaste noch besser aufeinander abgestimmt werden. Bei einer Weiterbildung des Ablaßventils nach Anspruch 17 schließt das Ablaßventil auch dann zuverlässig ab, wenn größere Fertigungs- und/oder Montagetoleranzen bei den einzelnen miteinander zusammenwirkenden Teilen auftreten. Diese werden dann von dem anfänglichen relativen Leerweg aufgefangen.

Für die Steuerung von kleinen Flüssigkeitsströmen, wie sie bei Infusionsgeräten auftreten, sind Schlauchventile bekannt (US PS 3,167,085 und US PS 3,460,526), bei denen ein Schlauch mit geschlossener Wandung an einem hohlzylinderförmigen Widerlager anliegt und von der Seite her mittels eines verstellbaren Druckkörpers mehr oder minder stark gegen das Widerlager gedrückt wird. Dabei wird der dem Druckkörper benachbarte Schlauchwandbereich in den Hohlraum des Schlauches hineinstülpt und schließlich mehr oder minder nahe an den am Widerlager anliegenden Wandbereich angenähert. Mit solchen Schlauchventilen kann aber nur bei Flüssigkeiten wegen deren größeren inneren Reibung der Mengenstrom einigermaßen gut gesteuert werden, und auch das nur, solange der Überdruck sehr gering ist und der Durchlaßquerschnitt nicht zu klein wird. Für Ablaßventile von Blutdruckmeßgeräten, bei denen Luft mit ihrer verhältnismäßig geringen inneren Reibung unter verhältnismäßig hohem Überdruck feinfühlig abgelassen werden muß, eignen sich diese Schlauchventile nicht. Da der eingestülpte Wandbereich praktisch auf seiner gesamten Umfangslänge entlang einer Halbkreislinie von dem am Widerlager anliegenden Wandbereich abgehoben wird, ändert sich der Durchlaßquerschnitt von Anfang an so schnell, und zwar ebenfalls näherungsweise nach einer quadratischen Funktion, daß dabei ein feinfühliges Verändern des Durchlaßquerschnittes nicht möglich ist. Gerade darauf kommt es aber bei Blutdruckmeßgeräten an.

Im folgenden wird die Erfindung anhand einiger in den Zeichnungen dargestellter Ausführungsbeispiele näher erläutert. Es zeigen:

Fig. 1     einen Längsschnitt eines ersten Ausführungsbeispieles des Ablaßventiles gemäß der Erfindung nach der Schnittsverlaufslinie I - I in Fig. 2;

Fig. 2     eine Draufsicht des Ablaßventils nach Fig. 1 bei abgeabgenommener Betätigungstaste;

Fig. 3     je einen Querschnitt des Ablaßventils nach

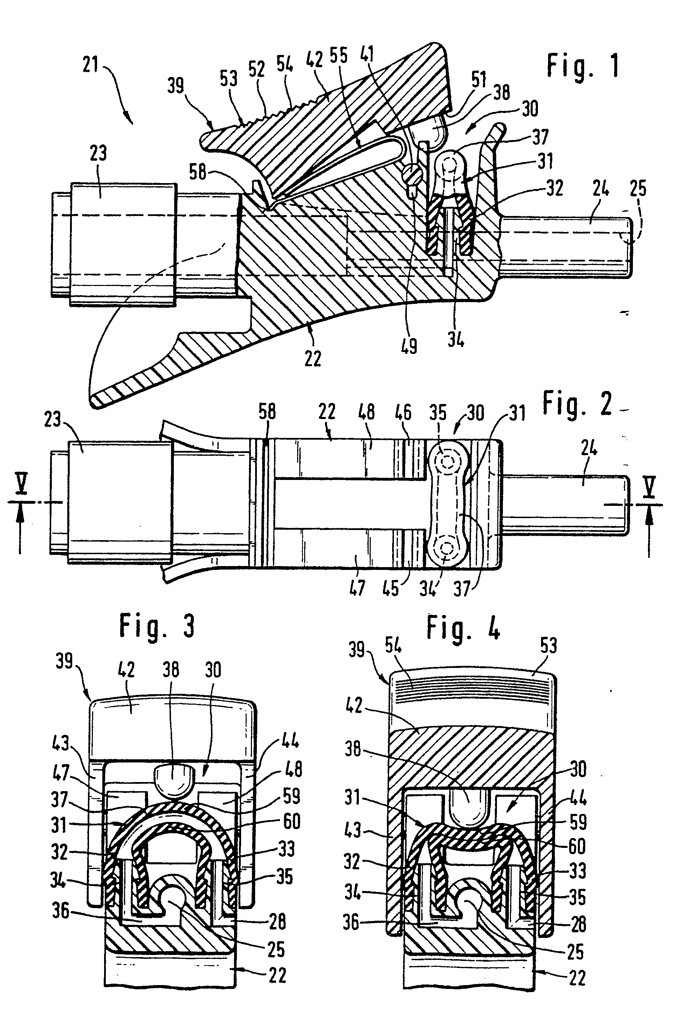

und 4     Fig. 1 in verschiedenen Betriebsstellungen;

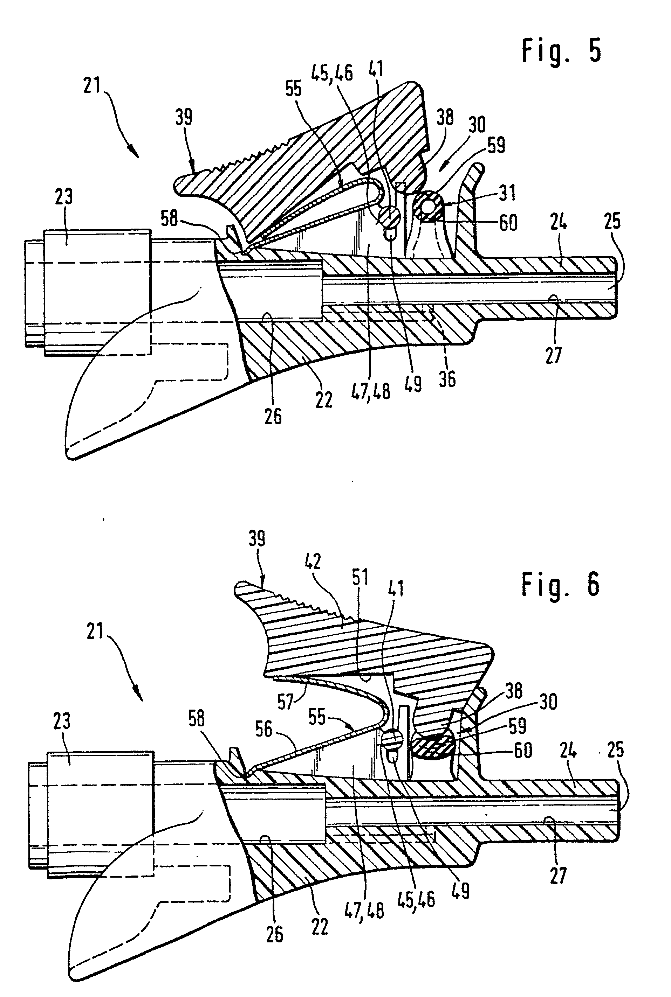

Fig. 5     je einen Längsschnitt des Ablaßventils nach

und 6     der Schnittverlaufslinie V - V in Fig. 2, in verschiedenen Betriebsstellungen;

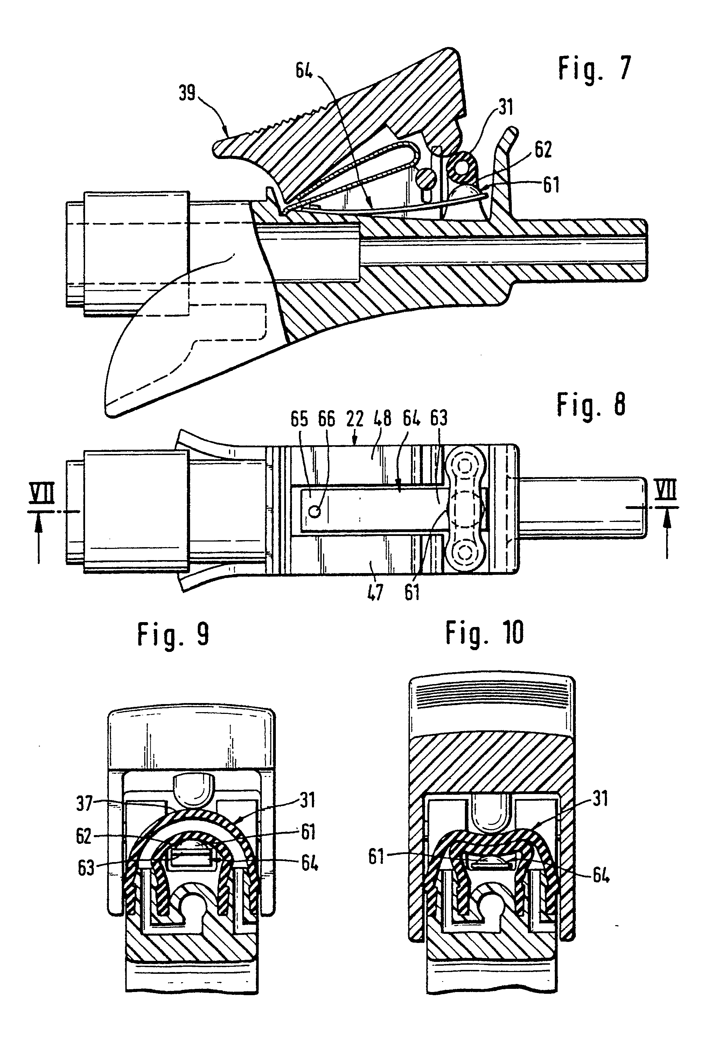

Fig. 7     einen Längsschnitt eines abgewandelten Ausführungsbeispiels des Ablaßventils nach der Schnittverlaufslinie VII - VII in Fig. 8;

Fig . 8     eine Draufsicht des Ablaßventils nach Fig. 7 bei abgenommener Betätigungstaste;

Fig. 9     je einen Querschnitt des Ablaßventils nach

und 10     Fig. 7 in verschiedenen Betriebsstellungen;

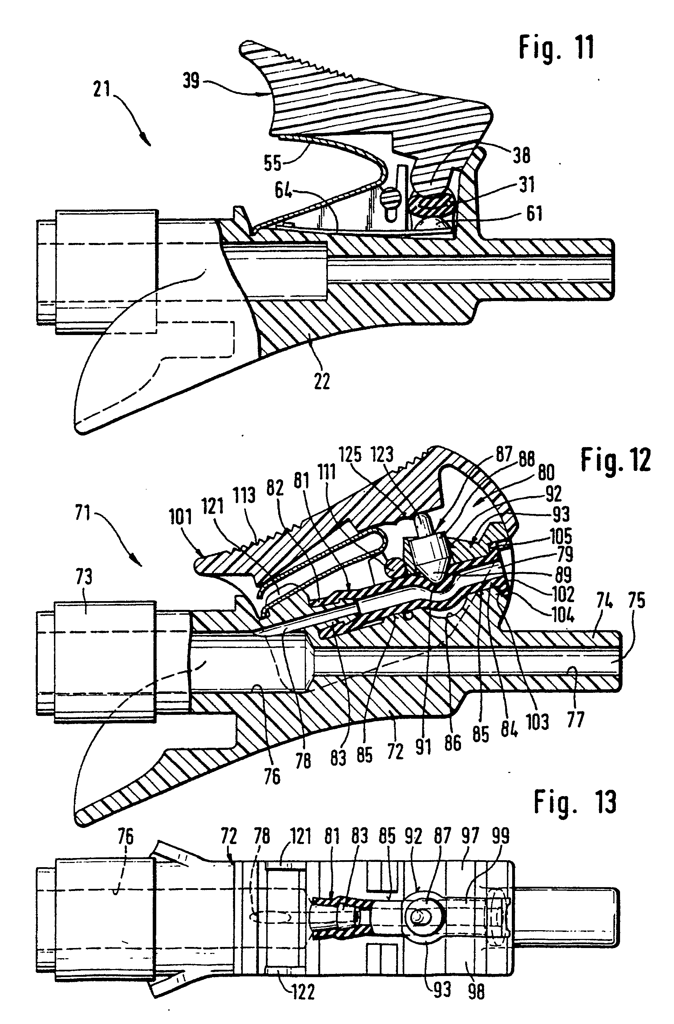

Fig. 11     einen Längsschnitt des Ablaßventils nach Fig. 7 in einer anderen Betriebsstellung;

Fig. 12     einen Längsschnitt eines zweiten Ausführungsbeispieles des Ablaßventils gemäß der Erfindung;

Fig. 13     eine Draufsicht des Ablaßventils nach Fig. 12 bei abgenommener Betätigungstaste;

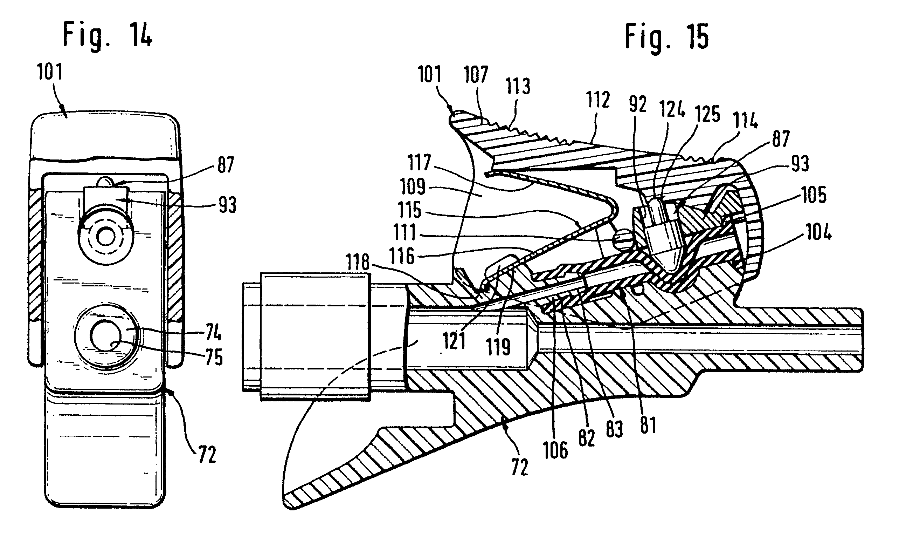

Fig. 14     eine Stirnansicht des Ablaßventils nach Fig. 12;

Fig. 15     einen Längsschnitt des Ablaßventils nach Fig. 12 in einer anderen Betriebsstellung;

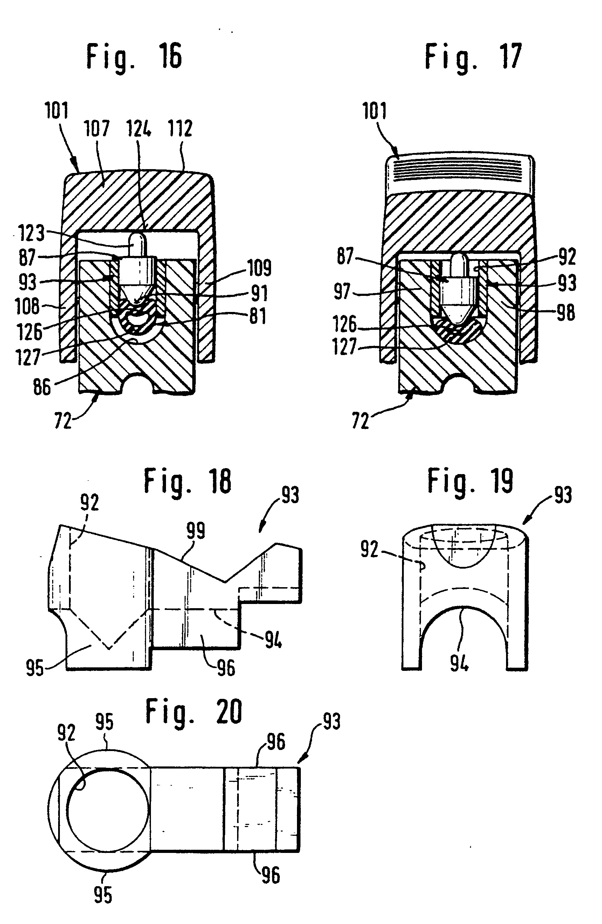

Fig. 16     je einen Querschnitt des Ablaßventils nach

und 17     Fig. 12 in verschiedenen Betriebsstellungen;

Fig. 18     verschiedene Ansichten eines Deckels für das

bis 20     Ablaßventil nach Fig. 12.

Bei dem aus Fig. 1 ... 6 ersichtlichen ersten Ausführungsbeispiel weist das Ablaßventil 21 ein Ventilgehäuse 22 auf, das ein Kunststoffspritzteil von näherungsweise quaderförmiger Gestalt ist. An jeder der beiden Stirnseiten ist je ein Anschlußstutzen angeformt. Der in Fig. 1 links gelegene Anschlußstutzen 23 dient dem Anschluß einer nicht dargestellten Gummiballpumpe. Der rechts gelegene Anschlußstutzen 24 dient dem Anschluß einer Verbindungsleitung in Form eines Druckschlauches, die das Ablaßventil 20 mit einer aufblasbaren Meßmanschette und meist zugleich auch mit einem Druckmeßgerät verbindet. Das Ventilgehäuse 22 weist im Inneren einen durchgehenden Luftkanal 25 auf, der durch die beiden Anschlußstutzen 23 und 24 hindurchgeht und an deren außenliegendem Ende mündet. Er hat zwei Längenabschnitte von unterschiedlicher Weite. Der vom Anschlußstutzen 23 ausgehende Kanalabschnitt 26 hat eine größere lichte Weite und der vom Anschlußstutzen 24 ausgehende Kanalabschnitt 27 hat eine kleinere lichte Weite. Das Ventilgehäuse 22 weist außerdem eine Auslaßöffnung 28 auf, die sich an der einen Längsseite befindet (Fig. 3 und 4). Zwischen dem Luftkanal 25 und der Auslaßöffnung 28 ist eine Dosiereinrichtung 30 eingeschaltet.

Die Dosiereinrichtung 30 weist einen kurzen Längenabschnitt eines Ventilschlauches 31 auf. Seine beiden Enden 32 und 33 sind auf je einen Schlauchstutzen 34 bzw. 35 aufgesteckt, die beide am Ventilgehäuse 22 angeformt sind. Beide Schlauchstutzen 34 und 35 sind auf der Oberseite des Ventilgehäuses 22 in der Querrichtung zu dessen Längserstreckung in einem gewissen gegenseitigen Abstand nebeneinander angeordnet. Ihr Außenabstand ist um mindestens die doppelte Wanddicke des Ventilschlauches 31 kleiner als die Breite des Ventilgehäuses 22. Die beiden Schlauchstutzen 34 und 35 sind zumindest annähernd parallel zueinander ausgerichtet.

Der Schlauchstutzen 34 steht über einen im Ventilgehäuse 22 eingeformten Verbindungskanal 36 mit dem Luftkanal 25 in Verbindung. Der Schlauchstutzen 35 steht mit der ins Freie führenden Auslaßöffnung 28 in Verbindung.

Wie aus Fig. 3 ersichtlich ist, ist der Ventilschlauch 31 zwischen den beiden Schlauchstutzen 34 und 35 in einem freien Bogen 37 geführt. Die Stichhöhe dieses freien Bogens ist etwa halb so groß wie der Abstand der Längsachsen der beiden Schlauchstutzen 34 und 35. Auf der von den Schlauchstutzen 34 und 35 abgekehrten Seite des Schlauchbogens 37 ist ein Druckkörper 38 an einer Schwenktaste 39 angeordnet. Der Druckkörper 38 ist an seinem Ende ballig, und zwar bevorzugt halbkugelförmig, ausgebildet.

Die Schwenktaste 39 bildet die Führung des Druckkörpers 38. Sie ist am Ventilgehäuse 22 mittels einer Schwenkachse 41 schwenkbar gelagert, die parallel zur Ebene des Schlauchbogens 37 ausgerichtet ist. Die Schwenktaste 39 hat einen U-förmigen Querschnitt (Fig. 3 und 4). Ihr Stegteil 42 ist näherungsweise plattenförmig ausgebildet und hat eine gewisse Dicke. Daran schließen die beiden dünneren Seitenwangen 43 und 44 an. Der lichte Abstand der beiden Seitenwangen 43 und 44 ist mindestens gleich, vorzugsweise geringfügig größer als die Breite des Ventilgehäuses 22. Die Schwenkachse 41 ist in einem gewissen Abstand zum Stegteil 42 als Verbindungsstab zwischen den beiden Seitenwangen 43 und 44 angeordnet und mit diesen fest verbunden, und zwar daran einstückig angeformt.

Die Schwenkachse 41 sitzt in zwei Schwenklagern 45 und 46, die auf der Oberseite des Ventilgehäuses 22 in je einer breiten Längsrippe 47 bzw. 48 mit parallelen Seitenwänden eingeformt sind. Die beiden Schwenklager 45 und 46 sind beide als zweischaliges Lager ausgebildet, dessen beide Lagerschalen durch einen durchgehenden Schlitz 49 voneinander getrennt sind.

Infolge dieses Schlitzes können die beiden Lagerschalen relativ zueinander federnd nachgeben, so daß bei der Montage der Schwenktaste ihre Schwenkachse 41 in die beiden Lagerschalen hineingedrückt werden kann, zwischen denen sie dann federnd einrastet.

Der Druckkörper 38 ist an der Unterseite 51 des Stegteils 42 der Schwenktaste 39 angeformt, und zwar in der Symmetrieebene der Schwenktaste. In der Längsrichtung sitzt der Druckkörper 38 auf der einen Seite der Schwenkachse 41, wobei seine Längsachse die Längsachse der Schwenkachse 41 in einem gewissen Abstand rechtwinklig kreuzt. Auf der in der Längsrichtung anderen Seite der Schwenkachse 41 hat der Stegteil 42 eine größere Längsausdehnung. Seine Oberseite 42 ist schwach zylindrisch gekrümmt, wobei die Zylindermantellinien quer zur Längserstreckung des Stegteils 42 ausgerichtet sind. Der von der Schwenkachse 41 entfernt gelegene Bereich der Oberseite 52 bildet die Tastfläche 53 der Schwenktaste 39. Diese ist vorzugsweise mit einer Querriffelung 54 versehen, wie es in Fig. 4 angedeutet ist. Anstelle oder zusätzlich zur Querriffelung 54 kann im Bereich der Tastfläche 54 die Oberseite 52 auch mit einer konkaven Tastmulde versehen sein.

In der Offenstellung des Ablaßventils 21 nimmt die Schwenktaste 39 die aus Fig. 1 und 5 ersichtliche Schwenkstellung ein. In der Schließstellung nimmt die Schwenktaste 39 die aus Fig. 6 ersichtliche Schwenkstellung ein. In diese Schwenkstellung wird die Schwenktaste 39 durch eine Blattfeder 55 gedrückt, die somit zugleich die Schließfeder des Ablaßventils 21 bildet. Die Blattfeder 55 ist U-förmig gebogen und weist die beiden Schenkel 56 und 57 auf. Der eine Schenkel 56 ist am Ende leicht abgebogen. Dieses abgebogene Ende greift in eine am Ventilgehäuse 22 dafür vorgesehene Ausnehmung 58 ein. Der andere Schenkel 57 liegt an der Unerseite 51 des Stegteils 42 der Schwenktaste 39 lose an, wobei er in seitlicher Richtung durch die Seitenwangen 43 und 44 der Schwenktaste geführt wird.

Da die Blattfeder 55 mit einer gewissen Vorspannung eingesetzt wird, genügt die einseitige Halterung an der Ausnehmung 58, um sie zwischen der Schwenktaste 39 und dem Ventilgehäuse 22 zuverlässig festzuhalten.

Die Schwenktaste 39 wird in ihrer Offenstellung (Fig. 1 und 5) durch eine im einzelnen nicht dargestellte Rastvorrichtung festgehalten, zu der an beiden Längsseiten des Ventilgehäuses 22 je eine Rastkerbe und an der Innenseite einer jeden Seitenwange 43 und 44 der Schwenktaste 39 (und zwar in dem von der Schwenkachse 48 entfernt gelegenen Bereich) je eine Rastleiste oder Rastrippe gehören, die so ausgebildet und angeordnet sind, daß sie gerade in der Offenstellung der Schwenktaste 39 ineinander greifen.

In der Offenstellung des Ablaßventils 21 ist der Ventilschlauch 31 zwischen den beiden Schlauchstutzen 34 und 35 als Bogen 37 frei gespannt (Fig. 3). Wenn die Schwenktaste 39 aus ihrer Rastvorrichtung gelöst wird und sie durch die Blattfeder 55 in die Schließstellung verschwenkt wird, drückt der Druckkörper 38 den Schlauchbogen 37 in der Bogenebene so weit ein, bis er in Bezug auf die ursprüngliche Bogenkrümmung einwärts verformt ist (Fig. 4). Dabei wird am Ventilschlauch 31 der am Schlauchbogen 37 außen gelegene Wandbereich 59 auf Grund der balligen Anlagefläche des Druckkörpers 38 auch in seiner Querschnittsform bis zum umgekehrten Krümmungsverlauf eingebeult (Fig. 6). Im Zuge beider Verformungsvorgänge schmiegt der Wandbereich 59 sich an den am Schlauchbogen 37 innen gelegenen Wandbereich 60 eng an und schließt an dieser Stelle den Hohlraum des Ventilschlauchs 31 vollständig ab (Fig. 4 und 6). Wenn die Betätigungstaste 39 entgegen der Schließkraft der Blattfeder 55 in Richtung auf ihre Offenstellung hin betätigt wird, folgt die eingebeulte Stelle des Ventilschlauches 31 dem Druckkörper 38 elastisch nach. Während des ersten Ab- schnittes der Aufrichtbewegung des Ventilschlau- ches 31 bleibt der Hohlraum noch verschlossen. Im weiteren Verlauf der Aufrichtbewegung des Ventilschlauches 31 öffnet sich der Hohlraum ganz allmählich, weil auch der unten gelegene Wandbereich 60 immer noch elastisch der Rückzugsbewegung des Druckkörpers 38 folgt. Erst nach einer gewissen Wegstrecke der Rückzugsbewegung des Druckkörpers 38 bleibt der untere Wandbereich 60 zunehmend stärker hinter dem oberen Wandbereich 59 zurück, so daß der Durchlaßquerschnitt des Hohlraums schneller zunimmt und dadurch dann eine Schnellentlüftung des gesamten zusammenhängenden Luftvolumens der Meßmanschette, der Verbindungsleitungen und des Ablaßventils 21 eintritt.

Aus Fig. 7 ... 11 ist das Ablaßventil 21 in einer durch einen Rückführkörper 61 geringfügig abgewandelten Ausführungsform ersichtlich. Die übrigen Teile des Ablaßventils 21 sind die gleichen wie zuvor.

Der Rückführkörper 61 ist unterhalb des Schlauchbogens 37 angeordnet. Er hat auf der dem Ventilschlauch 31 zugekehrten Seite eine ballige Anlage fläche. Er ist am freien Ende 63 einer Feder 64 befestigt. Die Blattfeder 64 liegt in dem rinnenförmigen Innenraum zwischen den beiden Rippen 47 und 48 auf der Oberseite des Ventilgehäuses 22. Ihr zweites Ende 65 liegt am Boden dieser Rinne auf und ist dort mittels eines Schweißzapfens 66 befestigt, der für diese Abwandlung des Ablaßventils 21 am Ventilgehäuse 22 angeformt ist.

Die Blattfeder 64 ist so geformt, daß sie in der Offenstellung der Schwenktaste 39 (Fig. 7) noch eine gewisse Restspannung aufweist. Wenn die Schwenktaste 39 in die Schließstellung verschwenkt wird (Fig. 11) und ihr Druckkörper 38 den Ventilschlauch 31 zusammendrückt und zum Ventilgehäuse 22 hin niederdrückt, dann weicht der unterhalb des Ventilschlauchs 31 liegende Rückführkörper 61 mit der Blattfeder 64 elastisch aus, wobei die Spannkraft der Blattfeder 64 sich erhöht. Wenn die Schwenktaste 39 aus der Schließstellung herausgeschwenkt und mehr oder minder weit in Richtung auf ihre Offenstellung hin verschwenkt wird, sorgt der Rückführkörper 68 unter der Wirkung der Rückstellkraft der Blattfeder 64 dafür, daß der Ventilschlauch 31 dem Druckkörper 38 sofort folgt und sich in dem Maße wieder aufrichtet, wie es der Druckkörper 38 zuläßt.

Anstelle des am Ventilgehäuses 22 sich abstützenden Rückführkörpers 61 kann das Aufrichten des Schlauchbogens 37 auch dadurch unterstützt werden, daß an der Schwenktaste 39 ein Arm befestigt oder gar angeformt wird, der sich bis auf die Unterseite des Schlauchbogens 37 erstreckt und in der aufgerichteten Stellung des Schlauchbogens 37 an dessen Unterseite lose anliegt. In der zusammengedrückten Stellung des Schlauchbogens 37 liegt dieser Arm dann zwar nicht an. Sobald die Schwenktaste 39 aber in etwas stärkerem Maße aus der Schließstellung zur Offenstellung hin verschwenkt wird, greift dieser Arm dann an der Unterseite des Schlauchbogens an, wenn dieser der Rückzugsbewegung des Druckkörpers 38 nicht sofort folgen sollte.

Im folgenden wird anhand Fig. 12 ... 20 als zweites Ausführungsbeispiel das Ablaßventil 71 erläutert. Soweit dabei einzelne Bauteile oder Bauteilgruppen nicht im einzelnen beschrieben werden, ist davon auszugehen, daß sie gleich oder zumindest ähnlich den entsprechenden Bauteilen oder Bau- teilgruppen des ersten Ausführungsbeispieles aus- gebildet sind.

Das Ventilgehäuse 72 weist wieder zwei Anschlußstutzen 73 und 74 auf, die dem Anschluß einer Gummiballpumpe bzw. eines Verbindungsschlauches dienen. Im Innern des Ventilgehäuses 72 befindet sich der durchgehende Luftkanal 75 mit den beiden unterschiedlich weiten Kanalabschnitten 76 und 77. Am Kanalabschnitt 76 zweigt in spitzem Winkel ein Auslaßkanal 78 ab, der in der Auslaßöffnung 79 mündet.

Die im Bereich des Auslaßkanals 78 angeordnete Dosiereinrichtung 80 weist einen kurzen Längenabschnitt eines Ventilschlauches 81 auf. Dessen eines Ende 82 ist auf einen Schlauchstutzen 83 aufgesteckt, der in der Fluchtlinie des Auslaßkanals 78 am Ventilgehäuse 72 in etwa in dessen Mittelebene oder Symmetrieebene angeformt ist. Die Mündung des zweiten Endes 84 des Ventilschlauches 81 bildet die Auslaßöffnung 79 des Ablaßventils 71.

In einer gewissen Entfernung vom Schlauchstutzen 83 beginnt eine zylindrische Rinne 85 mit halbkreisförmiger Querschnittsfläche, die an der Oberseite des Ventilgehäuses 72 angeformt ist und die auf den Ventilschlauch 81 abgestimmt ist. Im Bereich der Rinne 85 ist eine Vertiefung 86 vorhanden (Fig. 12 und 16). Sie ist als Rotationsfläche, insbesondere als Hohlkugelfläche, ausgebildet, deren Rotationsachse die Zylinderachse der kreiszylindrischen Anlagefläche der Rinne 85 rechtwinklig schneidet. Die lichte Weite der Vertiefung 86 ist mindestens gleich der halben Umfangslänge des Ventilschlauches 81.

Auf der von der Vertiefung 86 abgekehrten Seite des Ventilschlauches 81 ist ein Druckkörper 87 vorhanden. Er ist als Rotationskörper ausgebildet. Er weist einen zylindrischen Führungsteil 88 und einen zum Teil kegeligen Kopf 89 auf, der an der Spitze abgerundet ist und eine ballige, insbesondere kugelförmige Anlagefläche 91 für den Ventilschlauch 81 aufweist. Die Rotationsachse des Druckkörpers 87 einschließlich seines Führungsteils 88 und seiner Anlagefläche 91 sind mittig zur Rotationsachse der Vertiefung 86 angeordnet und ausgerichtet. Die Anlagefläche 91 des Druckkörpers 87 ist zumindest annähernd als Äquidistante zur Oberfläche der Vertiefung 86 ausgebildet, die bei völlig in die Vertiefung 86 eingedrücktem Ventilschlauch 81 (Fig. 15) gegenüber dieser einen Abstand von zumindest annähernd der doppelten Wanddicke des Ventilschlauches 81 hat.

Der Druckkörper 87 ist in einer Längsführung 92 mit kreiszylindrischer Führungsfläche geführt, die auf den Durchmesser des zylindrischen Führungsteils 88 am Druckkörper 87 abgestimmt ist. Die Längsführung 92 ist an einen Deckel 93 für die Rinne 85 angeordnet, und zwar daran angeformt (Fig. 18...20). In der Längsrichtung des Ventilschlauches 81 beginnt der Deckel 93 in einen gewissen Abstand vom Schlauchstutzen 83 spätestens am Rand der Vertiefung 86. Er erstreckt sich von dort bis über das Schlauchende 84 mit der Auslaßöffnung 79 etwas hinaus. An seiner Unterseite weist er eine zylindrische Gegenfläche 94 zur Rinne 85 auf, die ebenfalls auf die Außenfläche des Ventilschlauches 81 abgestimmt ist. Die Seitenflächen des Deckels 93 haben je einen zylindrischen Flächenteil 95 und einen ebenen Flächenteil 96. Die zylindrischen Flächenteile 95 sind mittig zur Zylinderfläche der Längsführung 92 ausgerichtet. Sie haben einen Halbmesser, der gleich dem Halbmesser der Vertiefung 86 der Rinne 85 ist. Die beiden ebenen Flächenteile 96 haben einen Außenabstand der kleiner als der Außenabstand der zylindrischen Flächenteile 95 ist. Dadurch bilden die zylindrischen Flächenteile 95 zugleich Führungsflächen in der Längsrichtung des Deckels 93, die dafür sorgen, daß der Deckel 93 in der Längsrichtung stets genau den richtigen Sitz hat. Im Bereich der ebenen Flächenteile 96 wird der Deckel 93 seitlich dadurch zusätzlich geführt, daß er sich an der innengelegenen Seitenwand eines rippenartigen Wandteils 97 bzw. 98 anlegt, dieauf der Oberseite des Ventilgehäuses 72 beiderseits der Rinne 85 vorhanden sind und bis zu einer gewissen Höhe über der Rinne 85 ansteigen. An diesen Wandteilen 97 und 98 ist auch jeweils die Gegenfläche für die zylindrischen Flächenteile 95 am Deckel 93 angeformt.

Die Oberseite 99 des Deckels 93 hat von der Seite gesehen den gleichen Aufriß wie die beiden Wandteile 97 und 98. Er ist zumindest zum Teil an den Verlauf der Unterseite der Schwenktaste 101 angepaßt, wenn diese ihre Schließstellung einnimmt (Fig. 15).

Wie aus Fig. 12 ersichtlich ist, ist der Ventilschlauch 81 an seinem vom Ventilstutzen 83 abgekehrten zweiten Ende 84 mit einem umlaufenden Bund 102 versehen, der auf seiner Rückseite eine kreisringförmige ebene Anlagefläche 103 aufweist. Dafür ist am Ventilgehäuse 72 eine Gegenfläche 104 und am Deckel 93 eine Gegenfläche 105 vorhanden (Fig. 15). Diese beiden Gegenflächen 104 und 105 haben vom Ansatz 106 des Schlauchstutzens 83 einen Abstand, der größer ist, als der Abstand der Anlagefläche 103 des Bundes 102 von dem entgegengesetzten Ende 82 des Ventilschlauches 81.

Das Übermaß richtet sich nach der Elastizität des Ventilschlauches 81 und ist um so kleiner je größer die Elastizität des Ventilschlauches 81 ist. Bei geringerer Elastizität kann es bis zu 1,5 mm betragen.

Anstelle des starren Übermaßes kann der angestrebte Zweck, nämlich eine elastische Streckung des Ventilschlauches 81, auch dadurch erreicht werden, daß der Abstand der Anlagefläche 103 am Bund 102 des Ventilschlauches 81 gleich oder sogar etwas größer als der Abstand der Gegenflächen 104 und 105 vom Ansatz des Schlauchstutzens 83 ausgeführt wird und dafür eine Feder, bevorzugt eine als Druckfeder wirkende Schraubenfeder, zwischen die Anlagefläche 103 und ihre Gegenflächen 104 und 105 eingefügt wird.

Die Schwenktaste 101 hat ähnlich der Schwenktaste 39 des ersten Ausführungsbeispieles einen U-förmigen Querschnitt mit einem Stegteil 107 und Seitenwangen 108 und 109. Ihre Schwenkachse 111 ist rechtwinklig zur Längsachse des Ventilschlauches 81 ausgerichtet. Sie erstreckt sich zwischen den Innenseiten der beiden Seitenwangen 108 und 109, an denen sie einstückig angeformt ist. Auf der vom Druckkörper 87 abgekehrten Seite der Schwenkachse 111 weist die Schwenktaste 101 auf ihrer Oberseite eine leicht zylindrisch gekrümmte Tastfläche 112 auf, deren Zylindermantellinien parallel zur Schwenkachse 111 ausgerichtet sind. In einem Teilbereich der Tastfläche 112 ist eine Querriffelung 113 angeformt. Eine ähnliche Querriffelung 114 ist auf der anderen Seite der Schwenkachse 111 ebenfalls an der Oberseite der Schwenktaste angebracht.

Die Schwenktaste 101 wird wiederum durch eine U-förmige Blattfeder 115 in ihre Schließstellung gedrückt, deren einer Schenkel 116 auf der Oberseite des Ventilgehäuses 72 und deren anderer Schenkel 117 an der Unterseite des Stegteils 107 der Schwenktaste 101 anliegt. Das Ende des Schenkels 116 ist leicht abgewinkelt und greift in eine Ausnehmung 118 am Ventilgehäuse 72 ein. Der daran anschließende Teil des Schenkels 116 liegt in einer flachen Ausnehmung 121 an, die mit seitlichen Führungswänden 121 und 122 versehen ist (Fig. 13).

Der Druckkörper 87 weist an dem vom Ventilschlauch 81 abgekehrten Ende einen Nachführzapfen 123 mit einer balligen Anlagefläche 124 auf. An der Unterseite der Schwenktaste 101 ist im Bereich der Anlage des Nachführzapfens l23 entlang der relativen Bewegungsbahn der Anlagefläche 124 des Nachführzapfens 123 eine Nockenbahn 125 angeformt. Im Bereich derjenigen Stelle der Nockenbahn 125, die der Schließstellung des Druckkörpers 87 entspricht (Fig. 15), sowie in dem unmittelbar daran anschließenden Abschnitt der Nockenbahn 125 ist ihre Steigung so gering, daß die Öffnungsbewegung des Druckkörpers 87 innerhalb der elastischen Verformung des noch geschlossen bleibenden Ventilschlauches 81 liegt. Der weitere Teil der Nockenbahn 125 ist so gewählt, daß die Querschnittvergrößerung des Hohlraumes des Ventilschlauches 81 zunächst verhältnismäßig gering ist, später aber progressiv zunimmt.

Die aus Fig. 12 ersichtliche Offenstellung der Schwenktaste 101 ist zugleich ihre Ruhelage, in der sie durch die nicht dargestellte Rastvorrichtung festgehalten wird. Wenn sie aus dieser Ruhelage herausbewegt wird und ihr jenseits der Schwenkachse 111 gelegener Teil im Uhrzeigersinn schwenkt, wird der an der Nockenbahn 125 anliegende Nachführzapfen 123 und damit der Druckkörper 87 auf den Ventilschlauch 81 hingedrückt. Dabei wird der am Druckkörper 87 anliegende Wandbereich 126 des Ventilschlauches 81 (Fig. 16) in zunehmendem Maße eingebeult und zugleich dem gegenüberliegenden Wandbereich 127 genähert, der der Vertiefung 86 benachbart ist. Diese Einbeulung des Wandbereiches 126 erfolgt sowohl in der Längsschnittebene (Fig. 12 und 15) wie auch in der Querschnittsebene (Fig. 16). Spätestens dann, wenn diese Einbeulung den gegenüberliegenden Wandbereich 127 erreicht hat, tatsächlich jedoch wegen des überall schon früher, wird auch der Wandbereich 127 zumindest in der Längsschnittebene zunehmend ausgebeult, bis beide Wandbereiche 126 und 127 sich schließlich eng aneinander anschmiegen und sie dabei weit in die Vertiefung 86 hineinragen (Fig. 15 und 17). Diese räumliche Verformung des Wandbereiches 126 und die ihr folgende Verformung des Wandbereiches 127 bewirken einen vollständigen Verschluß des Hohlraumes des Ventilschlauches 81. Bei der Öffnungsbewegung der Schwenktaste 101 und des Druckkörpers 87 aus ihrer Schließstellung heraus (Fig. 15) folgen anfänglich die beiden aneinander anliegenden Wandbereiche 126 und 127 gemeinsam elastisch dem Druckkörper 87 nach. Danach beginnt der Hohlraum des Ventilschlauches 81 sich allmählich wieder zu öffnen, wobei diese Öffnungsbewegung später sehr stark zunimmt.

## Ansprüche

1.  Ablaßventil für Blutdruckmeßgeräte mit einem Ventilgehäuse (22), das einen Luftkanal (25) aufweist, der mit einer Verbindungsleitung zu einer Meßmanschette verbindbar ist, der mit einer Auslaßöffnung (28) in Verbindung steht und mit einer Dosiereinrichtung (30) zwischen dem Luftkanal (25) und der Auslaßöffnung (28),
    **dadurch gekennzeichnet,**
    daß die Dosiereinrichtung (30) einen kurzen Längenabschnitt eines Ventilschlauches (31) aufweist, dessen beiden Enden (32, 33) auf je einem Schlauchstutzen (34, 35) am Ventilgehäuse (22) aufgesteckt sind, daß der eine Schlauchstutzen (34) mit dem Luftkanal (25) in Verbindung steht, daß der andere Schlauchstutzen (36) mit der Auslaßöffnung (28) in Verbindung steht, daß beide Schlauchstutzen (34, 35) in einem gewissen gegenseitigen Abstand nebeneinander angeordnet sind und zumindest annähernd parallel zueinander ausgerichtet sind, daß der Ventilschlauch (31) zwischen den Schlauchstutzen (34, 35) in einem freien Bogen (37) geführt ist, daß auf der von den Schlauchstutzen (34, 35) abgewandten Seite des Schlauchbogens (37) ein Druckkörper (38) vorhanden ist, der am Ventilgehäuse (22) mittels einer Betätigungstaste (39) zumindest annähernd in der Symmetrieebene zwischen den beiden Schlauchstutzen (34, 35) auf den Ventilschlauch (31) hin und von ihm weg bewegbar geführt ist, daß der Druckkörper (38) unter der Wirkung einer auf den Ventilschlauch (31) hin gerichteten Schließkraft (55) steht und daß der Druckkörper (38) mittels einer Betätigungstaste (39) entgegen der Schließkraft vom Ventilschlauch (31) weg bewegbar ist.

2.  Ablaßventil nach Anspruch 1,
    **dadurch gekennzeichnet,**
    daß die mittlere Höhe des zwischen den Schlauchstutzen (34, 35) frei geführten Bogens (37) des Ventilschlauches (31) zumindest annähernd halb so groß wie der Abstand der Schlauchstutzen (34, 35) ist.

3.  Ablaßventil nach Anspruch 1 oder 2,
    **dadurch gekennzeichnet,**

daß der Druckkörper (38) auf der dem Ventilschlauch (31) zugekehrten Seite ballig, vorzugsweise halbkugelförmig, ausgebildet ist.

4. Ablaßventil nach einem der Ansprüche 1 bis 3, **dadurch gekennzeichnet,** daß der Druckkörper (38) an einer Schwenktaste (39) angeordnet ist, die am Ventilgehäuse (22) mittels einer Schwenkachse (41) schwenkbar gelagert ist, die parallel oder senkrecht zur Ebene des Bogens (37) des Ventilschlauches (31) ausgerichtet ist und die auf der vom Druckkörper (38) abgekehrten Seite der Schwenkachse (41) vorzugsweise mit einer leicht zylindrischen Tastfläche (55) oder einer konkaven Fingermulde versehen ist.

5. Ablaßventil nach Anspruch 4, **dadurch gekennzeichnet,** daß für die Erzeugung der Schließkraft eine Blattfeder (55) vorhanden ist, die zumindest näherungsweise U-förmig ausgebildet ist, deren einer Schenkel (56) zumindest teilweise in eine auf die Gestalt dieses Schenkels (56) abgestimmte Ausnehmung (58) auf der Oberseite des Ventilgehäuses (22) eingelegt ist und deren anderer Schenkel (57) auf der von dem Druckkörper (38) abgekehrten Seite der Schwenktaste (39) an einer von der Schwenkachse (41) entfernt gelegenen Stelle an der dem Ventilgehäuse (22) zugekehrten Unterseite (51) der Betätigungstaste (39) anliegt.

6. Ablaßventil nach einem der Ansprüche 1 bis 5, **dadurch gekennzeichnet,** daß auf der Innenseite des Schlauchbogens (37) ein Rückführkörper (61) vorhanden ist, der unter der Wirkung einer auf den Schlauchbogen (37) hin gerichteten Kraft (64) steht, wobei bevorzugt der Rückführkörper (61) mit einer Anlagefläche (62) für den Schlauch (31) versehen ist, die vorzugsweise auf die Anlagefläche des Druckkörpers (38) abgestimmt ist, und wobei der Rückführkörper (61) bevorzugt am freien Ende (63) einer am Ventilgehäuse (22) eingespannten Blattfeder (64) angeordnet oder angeformt ist.

7. Ablaßventil nach Anspruch 4 oder 5, **dadurch gekennzeichnet,** daß an der Schwenktaste (39), vorzugsweise auf derjenigen Seite der Schwenktaste (39), auf der sich der Druckkörper (38) befindet, ein Arm angeordnet ist, der sich bis auf die Innenseite des Schlauchbogens (37) des Ventilschlauches erstreckt und an diesem lose anliegt.

8. Ablaßventil für Blutdruckmeßgeräte mit einem Ventilgehäuse (22), das einen Luftkanal (25) aufweist, der mit einer Verbindungsleitung zu einer Meßmanschette verbindbar ist der mit einer Auslaßöffnung (28) in Verbindung steht und mit einer Dosiereinrichtung (30) zwischen dem Luftkanal (25) und der Auslaßöffnung, (28), **dadurch gekennzeichnet,** daß die Dosiereinrichtung (80) einen kurzen Längenabschnitt eines Ventilschlauches (81) aufweist, dessen eines Ende (82) auf einen Schlauchstutzen (83) aufgesteckt ist, der mit dem Luftkanal (75) in Verbindung steht, und dessen anderes Ende (84) die Auslaßöffnung (79) des Ablaßventils (71) bildet, daß der Ventilschlauch (81) in einem von dem Schlauchstutzen (83) entfernt gelegenen Längenbereich in einer Rinne (85) des Ventilgehäuses (72) liegt, die eine zylindrische Auflagefläche für den Ventilschlauch (81) aufweist, daß im Bereich der Rinne (85) eine Vertiefung (86) vorhanden ist, die als stetige Hohlfläche ausgebildet ist, daß auf der von der Vertiefung (86) abgekehrten Seite des Ventilschlauches (81) ein Druckkörper (87) vorhanden ist, der mittels einer am Ventilgehäuse (72) angeordneten Führung (92) im Bereich der Vertiefung (86) auf den Ventilschlauch (81) hin und von ihm wegbewegbar geführt ist, der unter der Wirkung einer auf die Vertiefung (86) hin gerichteten Schließkraft (115) steht und der mittels einer Betätigungstaste (101) entgegen der Schließkraft (115) vom Ventilschlauch (81) wegbewegbar ist, daß der Druckkörper (87) eine Anlagefläche (91) für den Ventilschlauch (81) aufweist, die auf die Hohlfläche der Vertiefung (86) abgestimmt ist.

9. Ablaßventil nach Anspruch 8, **dadurch gekennzeichnet,** daß die Hohlfläche der Vertiefung (86) als Rotationsfläche ausgebildet ist, deren Rotationsachse am theoretischen Durchstoßpunkt durch die zylindrische Auflagefläche der Rinne (83) wenigstens in der Querrichtung zumindest annähernd normal zur Hüllfläche der Rinne (85) ausgerichtet ist, und daß die Anlagefläche (91) des Druckkörpers (87) als konvexe Rotationsfläche ausgebildet ist, deren Rotationsachse wenigstens in der Schließstellung zumindest annähernd mit der Rotationsfläche der Vertiefung (86) fluchtet und als Äquidistante zur Hohlfläche der Vertiefung (86) ausgebildet ist, die bei völlig in die Vertiefung (86) eingedrücktem Ventilschlauch (81) von dieser einen Abstand hat, der zumindest annähernd gleich der doppelten Wanddicke des Ventilschlauches (81) ist.

10. Ablaßventil nach Anspruch 8 oder 9,
**dadurch gekennzeichnet,**
daß die Betätigungstaste als Schwenktaste (101) ausgebildet ist, deren Schwenkachse (111) bevorzugt entweder rechtwinklig oder parallel zur Längsachse des Ventilschlauches (81) ausgerichtet ist, daß der Druckkörper (87) auf der einen Seite der Schwenkachse (111) in der Bewegungsbahn der Unterseite der Schwenktaste (101) angeordnet ist, daß auf der anderen Seite der Schwenkachse (111) auf ihrer Oberseite vorzugsweise eine zylindrische Tastfläche (112) oder eine konkave Tastmulde vorhanden ist, und daß die Schwenktaste (101) bevorzugt unter der Wirkung einer U-förmigen Blattfeder (115) steht, die vorzugsweise auf der vom Druckkörper (87) abgekehrten Seite der Schwenkachse (111) angeordnet ist, wobei ihr einer Schenkel (116) zumindest zum Teil am Ventilgehäuse (72) in einer mit seitlichen Führungswänden (121, 122) versehenen Ausnehmung (119) anliegt oder in diese eingreift.

11. Ablaßventil nach Anspruch 10,
**dadurch gekennzeichnet,**
daß der Druckkörper mit der Unterseite der Schwenktaste fest verbunden ist, vorzugsweise daran angeformt ist, oder mit ihr verbindbar ist.

12. Ablaßventil nach einem der Ansprüche 8 bis 10,
**dadurch gekennzeichnet,**
daß der Druckkörper (87) in einer Längsführung (92) geführt ist, die vorzugsweise als Kreiszylinderfläche ausgebildet ist, deren Zylinderachse mit der Rotationsachse der Vertiefung (86) der Rinne (85) fluchtet, und daß der Druckkörper (87) als Rotationskörper ausgebildet ist.

13. Ablaßventil nach Anspruch 12,
**dadurch gekennzeichnet,**
daß die Längsführung (92) des Druckkörpers (87) an einem Deckel (93) für die Rinne (85) angeordnet ist, vorzugsweise daran angeformt ist, der außerhalb eines vom Schlauchstutzen (83) entfernt gelegenen Teils der Rinne (85) angeordnet ist und mit dem Ventilgehäuse (72), vorzugsweise abnehmbar, verbunden ist.

14. Ablaßventil nach einem der Ansprüche 8 bis 13,
**dadurch gekennzeichnet,**
daß der Ventilschlauch (81) an dem vom Schlauchstutzen (83) abgekehrten Ende (84) einen Vorsprung oder Rücksprung mit einer Anlagefläche (103) aufweist, der bevorzugt als ein am Schlauchende (84) angeformter umlaufender Bund (102) ausgebildet ist, und daß am Ventilgehäuse (72) und/oder an einem mit ihm verbundenen Teil (93) eine Gegenfläche (104, 105) für die Anlagenfläche (103) des Vorsprungs oder Rücksprungs, insbesondere des Bundes (102), vorhanden ist, die vom Ansatz (106) des Schlauchstutzens (83) am Ventilgehäuse (72) einen Abstand hat, der vorzugsweise um bis zu 1,5 mm größer ist als der Abstand der Anlagefläche (103) des Vorsprungs oder Rücksprungs, insbesondere des Bundes (102), von dem entgegengesetzten Ende (82) des Ventilschlauches (81).

15. Ablaßventil nach einem der Ansprüche 8 bis 13,
**dadurch gekennzeichnet,**
daß der Ventilschlauch (81) an dem vom Schlauchstutzen (83) abgekehrten Ende (84) einen Vorsprung oder Rücksprung mit einer Anlagenfläche (103) aufweist, der bevorzugt als ein am Schlauchende (84) angeformter umlaufender Bund (102) ausgebildet ist, und daß am Ventilgehäuse (72) und/oder an einem mit ihm verbundenen Teil (93) eine Gegenfläche (104, 105) für die Anlagefläche (103) des Vorsprungs oder Rücksprungs, insbesondere des Bundes (102), vorhanden ist, die vom Ansatz des Schlauchstutzens (106) am Ventilgehäuse (72) einen Abstand hat, der kleiner, gleich oder höchstens bis zu 1,5 mm größer als der Abstand der Anlagefläche (103) des Vorsprungs oder Rücksprungs, insbesondere des Bundes (102), von dem entgegengesetzten Ende (82) des Ventilschlauches (81) ist, und daß zwischen die Anlagefläche (103) am Ventilschlauch (81) und ihre Gegenfläche (104, 105) am Ventilgehäuse (72) und/oder an dem mit ihm verbundenen Teil (93) eine als Druckfeder wirkende Feder, bevorzugt in Form einer Schraubenfeder, eingefügt ist.

16. Ablaßventil nach einem der Ansprüche 12 bis 15,
**dadurch gekennzeichnet,**
daß an der Unterseite der Schwenktaste (101) im Bereich der Anlage für den Druckkörper (87) entlang der relativen Bewegungsbahn der Anlagefläche (124) am Druckkörper (87) eine Nockenbahn (125) vorhanden ist, die vorzugsweise an der Schwenktaste (101) angeformt ist, und daß die Steigung der Nockenbahn (125) von der der Schließstellung des Druckkörpers (87) entsprechenden Stelle aus in Richtung auf die der Offenstellung des Druckkörpers (87) entsprechenden Stelle hin progressiv zunimmt.

**17.** Ablaßventil nach Anspruch 16,
**dadurch gekennzeichnet,**
daß im Bereich derjenigen Stelle der Nockenbahn (125), die der Schließstellung des Druckkörpers (87) entspricht, und in dem unmittelbar
daran anschließenden Abschnitt der Nockenbahn (125) ihre Steigung gleich null ist oder so
gering ist, daß die Öffnungsbewegung des
Druckkörpers (87) innerhalb der elastischen
Verformung des noch geschlossen bleibenden
Ventilschlauchs (81) liegt.

## Claims

1. Discharge valve for blood pressure instruments
with a valve housing (22) which has an air duct
(25), which can be connected by a connecting
cable to a measuring cuff, which duct is connected to a discharge opening (28), and with a
dosing mechanism (30) between the air duct
(25) and the discharge opening (28), characterized in that the dosing mechanism (30) has a
short length of valve hose (31), each end (32,
33) of which is attached to a hose connecting
pipe (34, 35) on the valve housing (22), that
one hose connecting piece (34) is connected
to the air duct (25), that the other hose connecting piece (36) is connected to the discharge opening (28), that both hose connecting
pieces (34, 35) are arranged next to one another at a given mutual spacing and are
aligned at least approximately parallel to one
another, that the valve hose (31) is guided
between the hose connecting pieces (34, 35)
in a free arc (37), that there is a pressing
member (38) on the side of the hose arc (37)
turned away from the hose connecting pieces
(34, 35) which is guided towards the valve
hose (31) on the valve housing (22) by means
of an operating key (39) at least approximately
in the plane of symmetry between both hose
connecting pieces (34, 35) and can be moved
away from it, that the pressing member (38) is
under the influence of a closing force (55)
directed towards the valve hose (31) and that
the pressing member (38) can be moved away
from the valve hose against the closing force
by means of an operating key (39).

2. Discharge valve according to Claim 1, characterized in that the average height of the arc
(37) of the valve hose (31) guided freely between the hose connecting pieces (34, 35) is at
least approximately half as great as the distance between the hose connecting pieces (34,
35).

3. Discharge valve according to Claim 1 or 2,
characterized in that the pressing member (38)
on the side turned towards the valve hose (31)
is spherical, preferably hemispherical.

4. Discharge valve according to one of the Claims
1 to 3, characterized in that the pressing member (38) is arranged on a swivel key (39) which
is positioned pivotally on the valve housing
(22) by means of a swivel axis (41) which is
aligned parallel or perpendicular to the plane of
the arc (37) of the valve hose (31), and on the
side of the swivel axis (41) turned away from
the pressing member (38) preferably has a
slightly cylindrical surface (55 sic) or a concave finger depression.

5. Discharge valve according to Claim 4, characterized in that there is a leaf spring (55) for
creating the closing force, which is at least
approximately U-shaped, one leg (56) of which
is at least partially inserted into a recess (58)
which corresponds in shape to this leg (56) on
the upper side of the valve housing (22) and
whose other leg (57) lies against the side of
the swivel key (39) turned away from the
pressing member (38) at a place which lies
away from the swivel axis (41) on the underside (51) of the operating key (39) turned towards the valve housing (22).

6. Discharge valve according to one of the Claims
1 to 5, characterized in that there is a feedback
element on the inside of the hose arc (37)
which is under the influence of a force (64)
directed towards the hose arc (37), whereby
preferably the feedback element (61) has a
support surface (62) for the hose (31), which
preferably corresponds to the support surface
of the pressing member (38), and whereby the
feedback element (61) is preferably arranged
or moulded onto the free end (63) of a leaf
spring (64) which is attached to the valve housing (22).

7. Discharge valve according to Claim 4 or 5,
characterized in that an arm is arranged on the
swivel key (39), preferably on the side of the
swivel key (39) on which the pressing member
(38) is located, which extends to the inside of
the hose arc (37) of the valve hose and lies
loosely against this.

8. Discharge valve for blood pressure instruments
with a valve housing (22) which has an air duct
(25) which can be connected by a connecting
cable to a measuring cuff, which duct is connected to a discharge opening (28), and with a dosing mechanism (30) between the air duct (25) and the discharge opening (28), characterized in that the dosing mechanism (80) has a short length of valve hose (81), one end of which is attached to a hose connecting piece (83) which is connected to the air duct (75) and the other end of which (84) forms the discharge opening (79) of the discharge valve (71), that the valve hose (81) lies in a longitudinal area away from the hose connecting piece (83) in a groove (85) of the valve housing (72) which has a cylindrical support surface for the valve hose (81), that there is a depression (86) in the area of the groove (85) which is designed as a continuous concave surface, that there is a pressing member (87) on the side of the valve hose (81) turned away from the depression (81) which is guided towards the valve hose (81) by means of a guide (92) arranged on the valve housing (72) in the area of the depression (86) and can be moved away from this hose, which is under the influence of a closing force (115) directed towards the depression (86) and can be moved away from the valve hose (81) against the closing force (115) by means of an operating key (101), that the pressing member (87) has a support surface (91) for the valve hose (81) which corresponds with the concave surface of the depression (86).

9. Discharge valve according to Claim 8, characterized in that the concave surface of the depression (86) is designed as a surface of rotation whose axis of rotation at the theoretical point of intersection through the cylindrical support surface of the groove (83) at least in the transverse direction is directed at least approximately perpendicular to the envelope surface of the groove (85) and that the support surface (91) of the pressing member (87) is designed as a convex surface of rotation whose axis of rotation at least in the closed position is at least approximately aligned with the surface of rotation of the depression (86) and designed as equidistant to the concave surface of the depression (86), which when the valve hose (81) is completely inserted into the depression (86) is at a distance from this depression which is at least approximately equal to twice the wall thickness of the valve hose (81).

10. Discharge valve according to Claim 8 or 9, characterized in that the operating key is designed as a swivel key (101) whose swivel axis (111) is preferably designed either perpendicular or parallel to the longitudinal axis of the valve hose (81), that the pressing member (87) on one side of the swivel axis (111) is arranged in the path of motion of the underside of the swivel key (101), that there is preferably a cylindrical surface (112) or a concave depression on the other side of the swivel axis (111) on its upper side, and that the swivel key (101) is preferably under the influence of a U-shaped leaf spring (115) which is preferably arranged on the side of the swivel axis turned away from the pressing member (87), one of its legs (116) lying in a recess (119) which has lateral guide walls (121, 122) or engaging in these.

11. Discharge valve according to Claim 10, characterized in that the pressing member is securely attached to the underside of the swivel key, preferably moulded onto it or connectable with it.

12. Discharge valve according to one of the Claims 8 to 10, characterized in that the pressing member (87) is guided in a longitudinal guide (92) which is preferably designed as a circular cylindrical surface whose cylindrical axis is aligned with the axis of rotation of the depression (86) of the groove (85) and that the pressing member (87) is designed as a body of rotation.

13. Discharge valve according to Claim 12, characterized in that the longitudinal guide (92) of the pressing member (87) is arranged on a lid (93) for the groove (85), preferably moulded onto it, which is arranged outside one of the parts of the groove (85) at a distance from the hose connecting piece (83) and is connected, preferably removably, to the valve housing (72).

14. Discharge valve according to one of the Claims 8 to 13, characterized in that the valves hose (81) has a projection or a depression with a support surface (103) at the end turned away from the hose-connecting piece (83) which is preferably designed as a rotating collar moulded onto the end of the hose (84), and that there is a complimentary surface (104, 105) to the support surface (103) of the projection or depression, particularly of the collar, on the valve housing (72) and/or on a part connected to it (93), which is at a distance from the lip of the hose connecting piece (83) on the valve housing (72) which is preferably up to 1.5 mm greater than the distance of the support surface (103) of the projection or depression, particularly of the collar (102), from the opposite end (82) of the valve hose (81).

15. Discharge valve according to one of the Claims 8 to 13, characterized in that the valve hose (81) has a projection or depression with a support surface (103) at the end (84) turned away from the hose connecting piece (83) which is preferably designed as a rotating collar moulded onto the hose end (84), and that there is a complementary surface (104, 105) to the support surface (103) of the projection or depression, particularly of the collar (102), on the valve housing (72) and/or on a part connected with it (93), which is at a distance from the lip of the hose connecting piece (106) on the valve housing (72) whic is smaller, equal to or a maximum of up to 1.5 mm greater than the distance of the support surface (103) of the projection or depression, particularly of the collar (102), from the opposite end (82) of the valve hose (81), and that between the support surface (103) on the valve hose (81) and its complementary surface (104, 105) a spring is attached on the valve housing (72) and/or on the part connected with it (93) which acts as a pressure gauge, preferably in the form of a screw spring.

16. Discharge valve according to one of the Claims 12 to 15, characterized in that there is a cam web (125) on the underside of the swivel key (101) in the support area for the pressing member (87) along the relative path of motion of the support surface (124) on the pressing member (87), which is preferably moulded onto the swivel key (101), and that the incline of the cam web (125) increases progressively from the place which corresponds to the closed position of the pressing member (87) to the place which corresponds to the open position of the pressing member (87).

17. Discharge valve according to Claim 16, characterized in that in the area of the place on the cam web (125) which corresponds to the closed position of the pressing member (87) and in the section of the cam web (125) directly adjacent to it, its decline is equal to zero or is so small that the opening movement of the pressing member (87) lies within the elastic deformation of the still closed valve hose (81).

**Revendications**

1. Soupape d'évacuation pour appareils de mesure de la tension artérielle comportant un boîtier (22) muni d'un canal d'air (25) qui peut être relié à une conduite de liaison d'un manchon de mesure, et communique avec un orifice d'échappement (28), un organe d'étranglement (30) étant prévu entre le canal d'air (25) et l'orifice d'échappement (28), caractérisée en ce que le dispositif de dosage (30) se compose d'un segment de courte longueur d'un tuyau (31) formant soupape, dont les deux extrémités (32, 33) sont emmanchées chacune sur un ajutage (34, 35) du boîtier de soupape (22), l'un des ajutages (34) communiquant avec le canal d'air (25) et l'autre ajutage (36) communiquant avec l'orifice de sortie (28), les deux ajutages (34, 35) étant séparés l'un de l'autre d'une certaine distance et sont alignés au moins de manière sensiblement parallèle, le tuyau (31) formant un arc libre (37) entre les ajutages (34, 35), et sur le côté de l'arc de cercle (37) opposé aux ajutages (34, 35), il est prévu un organe de pression (38) qui peut être rapproché ou écarté du tuyau (31), sur le boîtier (22) à l'aide d'une poignée de commande (39), au moins sensiblement dans le plan de symétrie entre les deux ajutages (34, 35), l'organe de pression (38) étant soumis à l'action d'une force de fermeture (55) dirigée vers le tuyau (31) et l'organe de pression (38) peut être actionné par une poignée de commande (39) contre la force de fermeture, pour s'écarter du tuyau (31).

2. Soupape d'évacuation selon la revendication 1, caractérisée en ce que la hauteur moyenne de l'arc libre (37) entre les ajutages (34, 35) du tuyau (31) est au moins approximativement égale à la moitié de la distance des ajutages (34, 35).

3. Soupape d'évacuation selon la revendication 1 ou 2, caractérisée en ce que l'organe de pression (38) présente une forme bombée et de préférence hémisphérique sur son côté opposé au tuyau (31).

4. Soupape d'évacuation selon l'une des revendications 1 à 3, caractérisée en ce que l'organe de pression (38) est prévu sur une poignée pivotante (39) qui est montée pivotante par l'intermédiaire d'un axe (41) sur le boîtier (22), cet axe étant parallèle ou perpendiculaire au plan de l'arc (37) du tuyau (31) et le côté de l'axe (41) opposé à l'organe de pression (38) est muni de préférence d'une surface de préhension (55) légèrement cylindrique ou d'une cavité pour doigt.

5. Soupape d'évacuation selon la revendication 4, caractérisée en ce que pour créer la force de fermeture, il est prévu un ressort-lame (55) qui présente au moins approximativement une forme de U, dont une branche (56) est au moins partiellement appliquée dans une cavité (58) de forme correspondant à celle de la branche (56), sur le côté supérieur du boîtier (22) et dont l'autre branche (57) s'applique du côté de la poignée (39) opposé à l'organe de pression (38) contre un point éloigné de l'axe de pivotement (41) de la face inférieure (51) de la poignée (39), face tournée vers le boîtier (22).

6. Soupape d'évacuation selon l'une des revendications 1 à 5, caractérisée par un organe de rappel (61) prévu sur le côté intérieur de l'arc de tuyau (37), organe qui est soumis à l'action d'une force (64) dirigée vers l'arc de tuyau (37), cet organe (61) étant de préférence muni d'une surface d'appui (62) pour le tuyau (31), surface qui correspond de préférence à la surface d'appui de l'organe de pression (38) et l'organe (61) est prévu ou est formé de préférence à l'extrémité libre (63) d'un ressort-lame (64) fixé au boîtier (22).

7. Soupape d'évacuation selon la revendication 4 ou 5, caractérisée par un bras prévu de préférence sur la poignée pivotante (39) et notamment sur le côté de cette poignée (39) où se trouve l'organe de pression (38), ce bras s'appliquant jusque contre le côté intérieur de l'arc (37) du tuyau et s'applique librement contre cet arc.

8. Soupape d'évacuation pour appareil de mesure de la tension artérielle comportant un boîtier de soupape (22) muni d'un canal d'air relié à une conduite de liaison d'un manchon de mesure en étant également relié à un orifice d'échappement (28) et à un dispositif de dosage (30) entre le canal d'air (25) et l'orifice d'échappement (28), caractérisée en ce que le dispositif de dosage (80) comporte un segment de longueur réduite d'un tuyau (81) dont une extrémité (82) est emmanchée sur un ajutage à tuyau (83) communiquant avec le canal d'air (75) et dont l'autre extrémité (84) forme l'orifice d'échappement (79) de la soupape d'échappement (71), le tuyau (81) étant placé avec sa partie longitudinale éloignée de l'ajutage (83) dans une goulotte (85) du boîtier (72), cette goulotte ayant une surface d'appui cylindrique pour le tuyau (81) et au niveau de la goulotte (85), il est prévu une cavité (86) en forme de surface creuse continue, et du côté du tuyau (81) opposé à la cavité (86), il est prévu un organe de pression (87) qui peut être guidé alternativement vers le tuyau de soupape (81) ou en s'éloignant de celui-ci par l'intermédiaire d'un moyen de guidage (92) prévu sur le boîtier (72) au voisinage de la cavité (86), sous l'action d'une force de fermeture (115) dirigée vers la cavité (86) et en pouvant s'écarter du tuyau (81) sous l'action d'une poignée de commande (101 agissant dans le sens contraire de la force de fermeture (115), l'organe de pression (87) ayant une surface d'appui (91) pour le tuyau (81), surface choisie en fonction de la surface creuse de la cavité (86).

9. Soupape d'évacuation selon la revendication 8, caractérisée en ce eue la surface creuse de la cavité (86) est une surface de rotation dont l'axe de rotation passe par le point d'intersection théorique de la surface d'appui cylindrique de la goulotte (83), en étant au moins sensiblement perpendiculaire à la direction transversale pour la surface-enveloppe de la goulotte (85) et en ce que la surface d'appui (91) de l'organe de pression (87) est en forme de surface de rotation convexe dont l'axe de rotation se trouve au moins pour la position de fermeture, approximativement aligné sur l'axe de rotation de la cavité (86) et équidistante par rapport à la surface creuse de la cavité (86) qui, lorsque le tuyau (81) est complètement enfoncé dans la cavité (86) est distante de celle-ci d'une distance au moins approximativement égale au double de l'épaisseur de paroi du tuyau (81).

10. Soupape d'évacuation selon la revendication 8 ou 9, caractérisée en ce que la poignée de commande est une poignée pivotante (101) dont l'axe de pivotement (111) est de préférence soit perpendiculaire, soit parallèle à l'axe longitudinal du tuyau (81), l'organe de pression (87) étant prévu d'un côté de l'axe de pivotement (111) dans la trajectoire de la face inférieure de la poignée pivotante (101), et l'autre côté de l'axe de pivotement (111), sur sa face supérieure, il est prévu de préférence une surface de préhension (112) cylindrique ou une cavité de préhension et en ce que la poignée pivotante (101) est de préféfence soumise à l'action d'un ressort-lame (115) en forme de U prévu notamment du côté de l'axe (111) opposé à l'organe de pression (87) et l'une des branches (116) de ce ressort vient prendre au moins partiellement appui sur le corps (72) dans une cavité (119) comportant des parois de guidage latérales (121, 122) ou s'applique contre cette cavité.

11. Soupape d'évacuation selon la revendication 10, caractérisée en ce que l'organe de pression est relié solidairement à la face inférieure de l'organe de pression en étant de préférence moulé sur cet organe ou en pouvant être relié à celui-ci.

12. Soupape d'évacuation selon l'une des revendications 8 à 10, caractérisée en ce que l'organe de pression (87) est guidé dans un organe de guidage longitudinal (92) de préférence en forme de surface cylindrique circulaire dont l'axe de cylindre est aligné sur l'axe de rotation de la cavité (86) de la goulotte (85) et en ce que l'organe de pression (87) est en forme d'organe de révolution.

13. Soupape d'évacuation selon la revendication 12, caractérisée en ce que l'organe de guidage longitudinal (92) de l'organe de pression (87) est prévu sur un couvercle (93) de la goulotte (85) en étant de préférence formé sur ce couvercle prévu à l'extérieur d'une partie de la goulotte (85) éloignée de l'ajutage (83) et en étant relié de préférence de manière amovible au boîtier (72).

14. Soupape d'évacuation selon l'une des revendications 8 à 13, caractérisée en ce que le tuyau (81) est muni à son extrémité (84) opposée à l'ajutage (83), d'une partie en saillie ou d'une partie de retrait avec une surface d'appui (103) de préférence en forme de collet (102) formée à l'extrémité (84) du tuyau et en ce que le boîtier (72) et/ou une pièce (93) reliée au boîtier présentant une surface antagoniste (104, 105) pour la surface d'appui (103) de la partie en saillie ou de la partie en retrait notamment du collet (102) qui est distante de l'épaulement (106) de l'ajutage (83) du corps (72) d'une distance supérieure jusqu'à 1,5 mm à la distance entre la surface d'appui (103) de la partie en saillie ou de la partie en retrait notamment du collet (102) et de l'extrémité opposée (82) du tuyau (81).

15. Soupape d'évacuation selon l'une des revendications 8 à 13, caractérisée en ce que le tuyau (81) comporte à son extrémité (84) opposée à l'ajutage (83), une partie en saillie ou en retrait avec une surface d'appui (103) qui est de préférence réalisée sous la forme d'un collet (102) formé à l'extrémité (84) du tuyau et en ce que le boîtier de soupape (72) et/ou une partie (93) reliée à ce boîtier présentant une surface antagoniste (104, 105) pour la surface d'appui (103) de la partie en saillie ou de la partie en retrait notamment du collet (102), surface qui est distante de l'épaulement de l'ajutage de tube (106) du boîtier (72), inférieure, égale ou plus supérieure de 1,5 mm à la distance entre la surface d'appui (103) de la partie en saillie ou de la partie en retrait notamment du collet (102) et de l'extrémité opposée (82) du tuyau (81) et en ce qu'entre la surface d'appui (103) du tuyau (81) et sa surface antagoniste (104, 105) sur le boîtier (72) du tuyau et/ou une partie (93) reliée à celui-ci, on peut interposer un ressort fonctionnant comme un ressort de pression, ressort constitué de préférence par un ressort hélicoïdal.

16. Soupape d'évacuation selon l'une des revendications 2 à 15, caractérisée en ce que sur la face inférieure de la poignée pivotante (101) au niveau de l'appui de l'organe de pression (87), le long de la trajectoire relative de la surface d'appui (124), l'organe de pression (87) comporte un chemin de cames (125) formé de préférence sur la poignée pivotante (101) et en ce que la pente du chemin de cames (125) augmente progressivement à partir de la position correspondant à la position de fermeture de l'organe de pression (87), en direction de la position d'ouverture de l'organe de pression (87), en augmentant progressivement.

17. Soupape d'évacuation selon la revendication 16, caractérisée en ce qu'au niveau de l'emplacement du chemin de cames (125) qui correspond à la position de fermeture de l'organe de pression (87) et au niveau du segment directement adjacent du chemin de cames (125), sa pente est pratiquement nulle ou tellement faible que le mouvement d'ouverture de l'organe de pression (87) reste dans la plage de déformation élastique du tuyau (81) qui reste encore fermé.

Fig. 1

Fig. 2

Fig. 3

Fig. 4

Fig. 5

Fig. 6

17

Fig. 7

Fig. 8

Fig. 9

Fig. 10

Fig. 11

Fig. 12

Fig. 13

Fig. 14

Fig. 15

101

87

93

74

75

72

101   107   113   112   92   124   125   114   87

117   93

109   105

115

111   104

116

118

121   119

72   106   82   83   81

EP 0 159 041 B1

Fig. 16

101 107 124 112

123
87
93
108
126
127
86
72

109
91
81

Fig. 17

101

87
97
126
127
72

92
93
98

Fig. 18

92 99 93

95 94
96

Fig. 19

93
92
94

Fig. 20

92 95 96 93

95 96